# EUROPÄISCHE PATENTSCHRIFT

(11) **EP 0 500 602 B1**
(45) Veröffentlichungstag und Bekanntmachung des Hinweises auf die Patenterteilung: **29.03.1995**
(21) Anmeldenummer: 90916090.5
(22) Anmeldetag: 08.11.1990
(51) Int. Cl.: A61K 38/36, C07K 14/745

(54) **MITTEL ZUR NEUTRALISIERUNG DER TUMORZELLEN-ASSOZIIERTEN PROKOAGULANT-AKTIVITÄT**
AGENT FOR NEUTRALISING THE PROCOAGULANT ACTIVITY ASSOCIATED WITH TUMOR CELLS
AGENT DE NEUTRALISATION DE L'ACTIVITE PROCOAGULANTE ASSOCIEE AUX CELLULES TUMORALES

(30) Priorität: 11.11.1989 DE 3937607
(43) Veröffentlichungstag der Anmeldung: 02.09.1992
(73) Patentinhaber: BOEHRINGER INGELHEIM INTERNATIONAL GmbH, 55218 Ingelheim am Rhein (DE)
(72) Erfinder: REUTELINGSPERGER, Christiaan, NL-6211 JK Maastricht (NL)
(86) Internationale Anmeldenummer: EP9001864
(87) Internationale Veröffentlichungsnummer: WO9107187

(56) Entgegenhaltungen:
- WO-A-88/07576
- Kitasato Arch. of Exp. Med., vol. 60, No.3, 1987, H.Maruyama et al,pages 105-121
- The Journal of Immunology, vol.145, No.1, 1 July 1990, The American Association of Immunologists , (US), T. Sakata et al, pages 387-396

## Beschreibung

Gegenstand der vorliegenden Erfindung ist die Verwendung eines antikoagulierenden Proteins aus der Familie der Annexine als Mittel zur Vermeidung der Metastasierung von Tumoren.

Als besonderes Problem bei Tumorerkrankungen stellt sich die Ausbildung von Metastasen dar. Den Mechanismus dieses als Metastasierung bezeichneten Phänomens stellt man sich folgendermaßen vor:
Tumorzellen überschwemmen das Zirkulationssystem des Organismus (Intravaskulation) und werden im Blutstrom transportiert. Diese malignen Zellen interagieren mit den Plättchen und den Plasma-Gerinnungsfaktoren und aktivieren das hämostatische System. Die von Tumorzellen bekannte oberflächenabhängige Prokoagulant-Aktivität führt zur Bildung von Thrombin und schließlich zum Fibrinpolymer. Außerdem treten dieselben Zellen auch mit anderen Wirtszellen in Wechselwirkung. Anschließend setzen sich die von Plättchen-Aggregaten und Fibrinfäden umgebenen Tumorzellen in Kapillargefäßen fest. Durch die Ausbildung des Fibringespinstes um die Oberfläche der Tumorzellen werden zudem spezifische Tumor-Antigene verborgen, die sonst eine immunologische Reaktion hervorrufen würden, die die Zerstörung der Tumorzelle zur Folge hätte. Gerade diese Fähigkeit der Tumorzelle, sich selbst mit einem Fibrinnetzwerk zu umgeben, wird daher verantwortlich gemacht für die metastatische Aktivität dieser Zellen. Tumorenzyme greifen daraufhin die Blutgefäßwand proteolytisch an, wodurch die Tumorzellen das Zirkulationssystem verlassen können (Extravaskulation); Sekundärtumore entstehen.

Entscheidend bei diesem Mechanismus ist offensichtlich die Fähigkeit der zirkulierenden Tumorzellen, die Bildung eines Fibringespinstes anzuregen, das dazu dient, die Zelle im Kapillargefäß festzusetzen und/oder sich der Immunabwehr des Organismus zu entziehen. Falanga et al. (Biochemistry 24, 5558-5567 (1985) isolierten und charakterisierten ein spezielles Krebs-Prokoagulant, das in der Lage war, die Koagulation durch Aktivierung des Faktors X auszulösen. Auch andere Krebs-Prokoagulantien, die direkt Faktor X aktivieren, wurden isoliert. Eine Zusammenfassung der Untersuchungen auf dem Gebiet der Aktivierung des Gerinnungssystems durch Tumorzellen findet sich in Cancer Metastasis Reviews 3, 99-116 (1984).

Marujama et al. (Kitasato Arch. of Exp. Med. 60 (3), 105-121 (1987)) beschreiben ein sulfatiertes Polysaccharidgemisch aus dem braunen Seegras *Laminaria religiosa*. Dieses Gemisch besteht in der Hauptsache aus 24,6 % L-Fucose und 27,6 % verestertem Sulfat. Diesem Substanzgemisch wird eine Antitumoraktivität zugeschrieben.

Aufgabe der vorliegenden Erfindung war daher, einen Wirkstoff bereitzustellen, der gegen die Prokoagulant-Aktivität der Tumorzellen gerichtet ist.

Die Bildung von Fibrin ist der letzte Schritt einer Kaskade enzymatischer Reaktionen, bei denen Thrombin gebildet wird, das letztendlich Fibrinogen in Fibrin umwandelt. Verschiedene prokoagulante Reaktionen wie beispielsweise die Aktivierung von Prothrombin durch die Faktoren Xa und Va werden katalysiert durch Phospholipidoberflächen, an die die Koagulationsfaktoren binden. Nicht jede Art der Phospholipide vermag die Gerinnung zu stimulieren. Die Ladung der Phosholipidoberfläche scheint das Ausmaß des Einflusses zu bestimmen. Negativ geladene Phospholipide wie Phosphatidylserin haben eine hohe prokoagulatorische Wirkung.

Bei den Proteinen, die an Phospholipide binden und mit Phospholipidoberflächen abhängigen Prozessen interferieren, gibt es eine Familie, die in ihrer Bindung an Phospholipide Ca²⁺ abhängig sind.

Zu dieser Familie, die auch Annexine genannt wird, gehört neben Lipocortin I, Calpactin I, Protein II, Lipocortin III, p67-Calelectrin auch das Vascular Antikoagulant Protein (VAC) und IBC, PAP, PAP I, PP4, Endonexin II und Lipocortin V. Auch Derivate dieser Proteine weisen solche Eigenschaften auf.

Die gemeinsamen strukturellen Merkmale der Annexine sind wahrscheinlich die Grundlagen für ihre ähnlichen Ca²⁺ und Phospholipidbindungseigenschaften. Obwohl diese generelle Eigenschaft für alle Annexine gilt, besteht eine klare Individualität hinsichtlich ihrer Affinität zu Ca²⁺ und zu den verschiedenen Phospholipidarten.

Die physiologischen Funktionen der Annexine betreffen membranassoziierte Prozesse. Der grundlegende Mechanismus der gerinnungshemmenden Wirkung des VAC wurde als eine Hemmung der katalytischen Kapazität der Phospholipide durch die Bindung des VAC an ihre Oberfläche erkannt, wodurch die Bildung des gerinnungsfördernden Komplexes an ihrer Oberfläche verhindert wird.

Auch andere Annexine können die Blutgerinnung hemmen, doch scheint VAC der effektivste Inhibitor zu sein.

Überraschenderweise wurde festgestellt, daß Annexine, insbesondere VAC die Prokoagulant-Aktivität von Tumorzellen inhibieren.

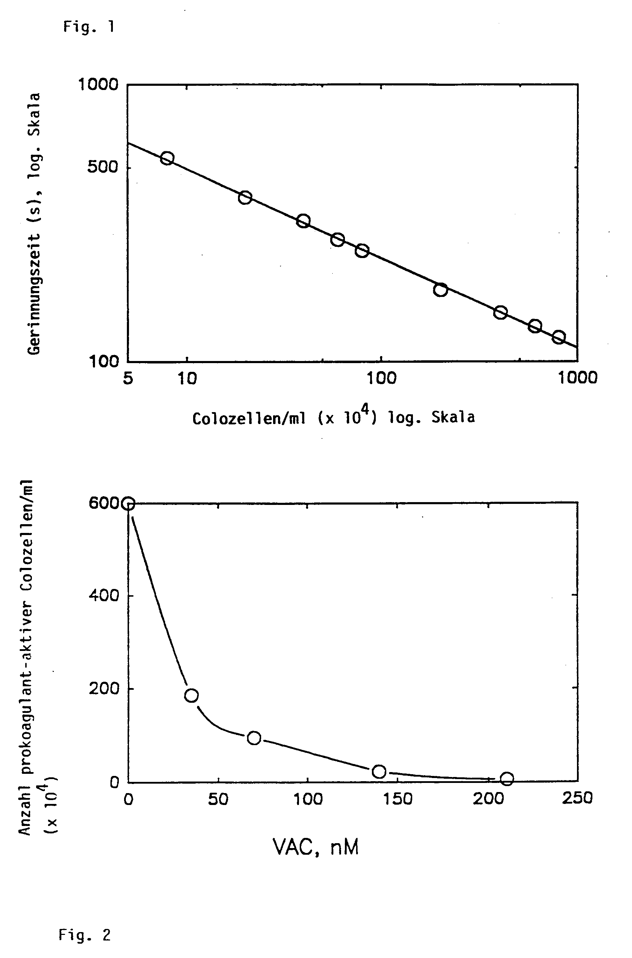

Die Prokoagulant-Aktivität-neutralisierende Wirkung der Annexine wurde an verschiedenen Colo-Tumorzellen nachgewiesen. Die Prokoagulant-Aktivität einer bestimmten Menge Colozellen wurde von VACα in dosisabhängiger Weise inhibiert (Fig. 2). Aus der Beziehung zwischen der Zellmenge und der Gerinnungszeit (Fig. 1) wurde errechnet, daß 210 nM VACα, 99% der Prokoagulant-Aktivität von 6x10⁶ Zellen/ml neutralisieren. Wurden durch VACα neutralisierte Colozellen in Gegenwart von Ca²⁺ zentrifugiert und anschließend mit TBSA gewaschen, zeigten sie keine Prokoagulant-Aktivität im Koagulationsassay. Enthielt die Flüssigkeit jedoch anstelle von Ca²⁺ EDTA, so zeigten die Zellen normale Prokoagulant-Aktivität.

Diese Ergebnisse deuten darauf hin, daß VACα die Tumorzellen assoziierte Prokoagulant-Aktivität neutralisiert, indem es Ca²⁺-abhängig an Oberflächenstellen bindet, die eine entscheidende Rolle für diese Prokoagulant-Aktivität spielen. Sehr wahrscheinlich sind also Phospholipide an der Tumorzellen assoziierten Prokoagulant-Aktivität beteiligt, da bisher keine anderen Oberflächenrezeptoren für VACα nachgewiesen wurden.

VACα inhibiert also die Prokoagulant-Aktivität von Tumorzellen, indem es an die Stellen der Oberfläche von Tumorzellen gebunden wird, die bei der Initiierung und/oder Propagierung der Reaktionen beteiligt sind, die zu Thrombin und letztendlich zur Fibrinbildung führen. Da neben VACα auch die anderen Annexine in Ca²⁺ abhängiger Weise an Phospholipide binden, dürften die mit VACα erhaltenen Ergebnisse auch auf diese übertragbar sein.

Dadurch, daß die Annexine die den Tumorzellen eigene Prokoagulant-Aktivität hemmen, verhindern sie die Einkapselung der Tumorzellen in ein Fibrinnetz; eine für die metastatische Aktivität der Tumorzellen unbedingte Notwendigkeit. Aufgrund der neutralisierenden Wirkung sind die Annexine daher in der Lage, die Metastasierung von Tumorzellen zu verhindern und stellen daher potente Wirkstoffe mit anti-metastatischer Aktivität dar. Als ein solcher Wirkstoff ist insbesondere VAC geeignet.

Die der Erfindung zugrunde liegende Aufgabe wird daher durch die Verwendung eines antikoagulierenden Proteins aus der Familie der Annexine gelöst.

Die erfindungsgemäß einsetzbaren Wirkstoffe, insbesondere VAC können nicht nur in freier Form, sondern auch in Form ihrer Salze, insbesondere ihrer pharmazeutisch annehmbaren Salze, vorliegen. Da sie mehrere Aminosäurereste mit freien Aminogruppen enthalten, können die erfindungsgemäßen Verbindungen z.B. in Form von Säureadditionssalzen vorliegen. Als Säureadditionssalze kommen insbesondere physiologisch verträgliche Salze mit üblichen, therapeutisch anwendbaren Säuren in Betracht; als anorganische Säuren sind die Halogenwasserstoffsäuren, wie die Chlorwasserstoffsäure, aber auch Schwefelsäure und Phosphor- bzw. Pyrophosphorsäure zu nennen; als organische Säuren sind in erster Linie Sulfonsäuren, wie die Benzol- oder p-Toluosulfonsäure oder Niederalkansulfonsäuren, wie Methansulfonsäure, sowie Carbonsäuren, wie Essigsäuren, Milchsäure, Palmitin- und Stearinsäure, Äpfelsäure, Weinsäure, Ascorbinsäure und Citronensäure geeignet. Da die Verbindungen auch Aminosäurereste mit freien Carboxylgruppen enthalten, können sie auch als Metallsalz, insbesondere als Alkalimetall- oder Erdalkalimetallsalz, z.B. Natrium-, Calcium- oder Magnesiumsalz, oder auch als Ammoniumsalz, abgeleitet von Ammoniak oder einer physiologisch verträglichen, organischen stickstoffhaltigen Base, vorliegen. Da sie aber zugleich freie Carboxylgruppen und freie Aminogruppen enthalten, können sie auch als inneres Salz vorliegen.

Die erfindungsgemäßen Mittel können z. B. parenteral, wie intravenös, intracutan, subcutan oder intramuskulär, oder topisch verabreicht werden.

Die Größe der Einzeldosen sowie das Verabreichungsschema kann am besten anhand einer individuellen Beurteilung des jeweiligen Krankheitsfalles bestimmt werden: die dazu erforderlichen Methoden zur Bestimmung von relevanten Faktoren sind dem Fachmann geläufig. Im Normalfall liegt bei einer Injektion die therapeutisch wirksame Menge der erfindungsgemäßen Arzneimittel im Dosisbereich von etwa 0,005 bis etwa 0,5 mg/kg Körpergewicht. Bevorzugt wird der Bereich von etwa 0,01 mg/kg bis etwa 0,1 mg/kg, besonders bevorzugt von etwa 0,01 bis etwa 0,05 mg/kg Körpergewicht. Die Verabreichung erfolgt durch intravenöse, intramuskuläre oder subcutane Injektion. Dementsprechend enthalten pharmazeutische Präparate zur parenteralen Verabreichung in Einzeldosis-Form in Abhängigkeit von der Applikationsart pro Dosis etwa 0,4 bis etwa 7,5 mg des Wirkstoffes. Die erfindungsgemäßen Arzneimittel enthalten neben den Wirkstoffen gegebenenfalls noch einen Puffer, z. B. einen Phosphatpuffer, der den pH-Wert zwischen etwa 3,5 und 8 halten soll, und ferner Natriumchlorid, Mannit oder Sorbit zur Einstellung der Isotonie. Sie können in gefriergetrockneter oder gelöster Form vorliegen, wobei Lösungen ein antibakteriell wirkendes Konservierungsmittel, z. B. 0,2 bis 0,3 % 4-Hydroxybenzoesäuremethylester oder -ethylester, enthalten können. Ein Präparat für die topische Anwendung kann als wäßrige Lösung, Lotion oder Gelee, ölige Lösung oder Suspension, oder fetthaltige oder insbesondere Emulsions-Salbe vorliegen. Ein Präparat in Form einer wäßrigen Lösung erhält man beispielsweise dadurch, daß man die erfindungsgemäßen Wirkstoffe in einer wäßrigen Pufferlösung von pH 4 bis 6,5 löst und gewünschtenfalls einen weiteren Wirkstoff, z. B. ein Antiinflammatorikum, und/oder ein polymeres Haftmittel, z. B. Polyvinylpyrrolidon, und/oder ein Konservierungsmittel zufügt. Die Konzentration des Wirkstoffs beträgt etwa 0,1 bis etwa 1,5 mg, vorzugsweise 0,25 bis 1,0 mg, in 10 ml einer Lösung bzw. 10 g eines Geles.

Eine ölige Applikationsform für die topische Verabreichung erhält man beispielsweise durch Suspendieren der erfindungsgemäßen Wirkstoffe in einem Oel, gegebenfalls unter Zusatz von Quellmitteln, wie Aluminiumstearat, und/oder grenzflächenaktiven Mitteln (Tensiden), deren HLB-Wert ("hydrophilic-lipophilic-balance") unter 10 liegt, wie Fettsäuremonoester mehrwertiger Alkohole, z. B. Glycerinmonostearat, Sorbitanmonolaurat, Sorbitanmonostearat oder Sorbitanmonooleat. Eine fetthaltige Salbe erhält man z.B. durch Suspendieren der erfindungsgemäßen Wirkstoffe in einer streichbaren Fettgrundlage, gegebenenfalls unter Zusatz eines Tensids vom HLB-Wert unter 10. Eine Emulsionssalbe erhält man durch Verreiben einer wäßrigen Lösung der erfindungsgemäßen Wirkstoffe in einer weichen, streichbaren Fettunterlage unter Zusatz eines Tensids, dessen HLB-Wert unter 10 liegt. Die topischen Applikationsformen können auch Konservierungsmittel enthalten. Die Konzentration des Wirkstoffes beträgt 0,1 bis 1,5 mg, vorzugsweise 0,25 bis 1,0 mg, in etwa 10 g der Grundmasse.

Gegenstand der vorliegenden Erfindung ist die:
Verwendung eines Antikoagulanz aus der Gruppe der Annexine und seiner natürlich vorkommenden oder synthetisch oder gentechnisch hergestellten Derivate oder Analoga zur Herstellung eines Arzneimittels zur Vermeidung der Metastasierung von Tumoren und

Verwendung eines Antikoagulanz aus der Gruppe der Annexine und seiner natürlich vorkommenden oder synthetisch oder gentechnisch hergestellten Derviate oder Analoga zur Herstellung eines Arzneimittels zur Vermeidung der Metastasierung, wobei das Antikoagulanz der Formel 
1                   5                        10                       15
Met Ala Gln Val Leu Arg Gly Thr Val Thr Asp Phe Pro Gly Phe

                        20                       25                       30
Asp Glu Arg Ala Asp Ala XX  Thr Leu Arg Lys Ala Met Lys Gly

                        35                       40                       45
Leu Gly Thr Asp Glu Glu Ser Ile Leu Thr Leu Leu Thr Ser Arg

                        50                       55                       60
Ser Asn Ala Gln Arg Gln Glu Ile Ser Ala Ala Phe Lys Thr Leu

                        65                       70                       75
Phe Gly Arg Asp Leu Leu Asp Asp Leu Lys Ser Glu Leu Thr Gly
 
80 85 90
Lys Phe Glu Lys Leu Ile Val Ala Leu Met Lys Pro Ser Arg Leu

95 100 105
Tyr Asp Ala Tyr Glu Leu Lys His Ala Leu Lys Gly Ala Gly Thr

110 115 120
Asn Glu Lys Val Leu Thr Glu Ile Ile Ala Ser Arg Thr Pro Glu

125 130 135
Glu Leu Arg Ala Ile Lys Gln Val Tyr Glu Glu Glu Tyr Gly Ser

140 145 150
Ser Leu Glu Asp Asp Val Val Gly Asp Thr Ser Gly Tyr Tyr Gln

155 160 165
Arg Met Leu Val Val Leu Leu Gln Ala Asn Arg Asp Pro Asp Ala

170 175 180
Gly Ile Asp Glu Ala Gln Val Glu Gln Asp Ala Gln Ala Leu Phe

185 190 195
Gln Ala Gly Glu Leu Lys Trp Gly Thr Asp Glu Glu Lys Phe Ile

200 205 210
Thr Ile Phe Gly Thr Arg Ser Val Ser His Leu Arg Lys Val Phe

215 220 225
Asp Lys Tyr Met Thr Ile Ser Gly Phe Gln Ile Glu Glu Thr Ile

230 235 240
Asp Arg Glu Thr Ser Gly Asn Leu Glu Gln Leu Leu Leu Ala Val

245 250 255
Val Lys Ser Ile Arg Ser Ile Pro Ala Tyr Leu Ala Glu Thr Leu
 
260 265 270
Tyr Tyr Ala Met Lys Gly Ala Gly Thr Asp Asp His Thr Leu Ile

275 280 285
Arg Val Met Val Ser Arg Ser Glu Ile Asp Leu Phe Asn Ile Arg

290 295 300
Lys Glu Phe Arg Lys Asn Phe Ala Thr Ser Leu Tyr Ser Met Ile

305 310 315
Lys Gly Asp Thr Ser Gly Asp Tyr Lys Lys Ala Leu Leu Leu Leu

320
Cys Gly Glu Asp Asp *
entspricht, wobei XX für Glu oder Asp steht und gegebenenfalls an Position 1 das Methionin abgespalten ist und das Alanin an Position 2 gegebenenfalls blockiert ist und/oder wobei gegebenenfalls Aggregationen durch beispielsweise intermolekulare Disulfidbrücken zwischen den Cysteinen an Position 316 vorliegen oder seinen biologisch aktiven Varianten oder Derivaten entspricht.

Das nachfolgende Beispiel soll die Erfindung erläutern.

### Materialien und Methoden

VACα wurde entweder analog EPA 0 181 465 oder EPA 0 293 567 hergestellt. Die nachfolgenden Untersuchungen wurden mit VACα durchgeführt. Die Ergebnisse dürften jedoch auch auf die anderen Annexine, insbesondere auf VACβ übertragbar sein.

Colo Tumorzellen wurden in Gewebekolben in RPMI 1640, supplementiert mit 10% fötalem Kälberserum kultiviert. Die Zellen wurden geerntet, indem der Boden des Kolbens mit einem Gummiwischer abgewischt wurde. Die gesammelten Zellen wurden mit einem TBSA-Puffer gewaschen (50 mM Tris/HCl, 100 mM NaCl, 4,5 mg Glukose/ml, 0,5 mg Bovines Serumalbumin/ml, 2 mM CaCl₂, pH 7,9) und schließlich für die Untersuchungen in diesem Puffer suspendiert. Die Lebensfähigkeit dieser Zellen wurde mit Tryptan-Blau Exklusion getestet. Mehr als 95% der Zellen waren nach dieser Prozedur lebensfähig.

Koagulations Assay: Die Prokoagulant-Aktivität der Colozellen wurde gemessen, indem eine bestimmte Anzahl gewaschener Zellen 50 »l zitriertem Plättchen-freiem-Plasma (PFP) zugegeben wurde. Diese Mischung wurde zwei Minuten bei 37°C gerührt. Koagulation wurde durch Zugabe von CaCl₂ (Endkonzentration war 10 mM) ausgelöst. Das Endvolumen der Mischung betrug 500 »l. Koagulation wurde turpidimetrisch mit einem "Payton dual wave aggregometer" beobachtet.

### Ergebnisse

### Colozellen assoziierte Prokoagulant-Aktivität: Wurden gewaschene Colozellen zitriertem PFP zugegeben und das Plasma wurde recalcifiziert, so konnte eine Gerinnungszeit beobachtet werden, die von der Anzahl der anwesenden Zellen abhing (Fig. 1). In Abwesenheit der Zellen betrug die Gerinnungszeit mehr als 15 Minuten, während 8x10⁶ Zellen/ml diese Gerinnungszeit auf etwa zwei Minuten reduzierte. Diese Prokoagulant-Aktivität wurde durch die intakten Zellen hervorgerufen; Zentrifugation der Zellen ergab eine Kopräzipitation der Prokoagulant-Aktivität.

### Neutralisation der Colozellen assoziierten Prokoagulant-Aktivität durch VACα: Die Prokoagulant-Aktivität einer bestimmten Menge Colozellen wurde von VACα in dosisabhängiger Weise inhibiert (Fig. 2). Aus der Beziehung zwischen der Zellmenge und der Gerinnungszeit (Fig. 1) wurde errechnet, daß 210 nM VACα 99% der Prokoagulant-Aktivität von 6x10⁶ Zellen/ml neutralisieren. Wurden durch VACα neutralisierte Colozellen in Gegenwart von Ca²⁺ zentrifugiert und anschließend mit TBSA gewaschen, zeigten sie keine Prokoagulant-Aktivität im Koagulationsassay. Enthielt die Flüssigkeit jedoch anstelle von Ca²⁺ EDTA, so zeigten die Zellen normale Prokoagulant-Aktivität.

### Legende zu den Figuren

### Fig. 1

Colozellen assoziierte Prokoagulant-Aktivität. Zitriertes Plättchen-freies-Plasma (PFP) wurde mit TBSA, enthaltend die angegebene Anzahl an Colozellen, verdünnt. Nach einer Inkubation von 3 Min. bei 37°C wurde CaCl₂ (Endkonzentration 10 mM) zugegeben und die Gerinnungszeit registriert.

### Fig. 2

Effekt von VACα auf die Prokoagulant-Aktivität von Colozellen. Zitriertes Plättchen-freies-Plasma (PFP) wurde mit TBSA, die angegebene Menge an VACα und eine bestimmte Anzahl an Colozellen enthaltend (bis zu einer Endkonzentration von 6x10⁶ Zellen/ml), verdünnt. Nach Inkubation von 3 Min. bei 37°C wurde die Koagulation durch Zugabe von CaCl₂ (Endkonzentration 10 mM) ausgelöst. Die gemessene Gerinnungszeit wurde bezogen auf Fig. 1 und auf die Anzahl von prokoagulant- aktiven Colozellen extrapoliert.

## Patentansprüche (Patentansprüche für folgende(n) Vertragsstaat(en): AT, BE, CH, DE, DK, FR, GB, IT, LI, LU, NL, SE)

1. Verwendung eines Antikoagulanz aus der Gruppe der Annexine und seiner natürlich vorkommenden oder synthetisch oder gentechnisch hergestellten Derivate oder Analoga zur Herstellung eines Arzneimittels zur Vermeidung der Metastasierung von Tumoren.

2. Verwendung eines Antikoagulanz gemäß Anspruch 1, wobei das Antikoagulanz der Formel 
1                   5                   10                  15
Met Ala Gln Val Leu Arg Gly Thr Val Thr Asp Phe Pro Gly Phe

                    20                  25                  30
Asp Glu Arg Ala Asp Ala XX  Thr Leu Arg Lys Ala Met Lys Gly

                    35                  40                  45
Leu Gly Thr Asp Glu Glu Ser Ile Leu Thr Leu Leu Thr Ser Arg

                    50                  55                  60
Ser Asn Ala Gln Arg Gln Glu Ile Ser Ala Ala Phe Lys Thr Leu

                    65                  70                  75
Phe Gly Arg Asp Leu Leu Asp Asp Leu Lys Ser Glu Leu Thr Gly

                    80                  85                  90
Lys Phe Glu Lys Leu Ile Val Ala Leu Met Lys Pro Ser Arg Leu

                    95                  100                 105
Tyr Asp Ala Tyr Glu Leu Lys His Ala Leu Lys Gly Ala Gly Thr

                    110                 115                 120
Asn Glu Lys Val Leu Thr Glu Ile Ile Ala Ser Arg Thr Pro Glu

                    125                 130                 135
Glu Leu Arg Ala Ile Lys Gln Val Tyr Glu Glu Glu Tyr Gly Ser

                    140                 145                 150
Ser Leu Glu Asp Asp Val Val Gly Asp Thr Ser Gly Tyr Tyr Gln

                    155                 160                 165
Arg Met Leu Val Val Leu Leu Gln Ala Asn Arg Asp Pro Asp Ala

                    170                 175                 180
Gly Ile Asp Glu Ala Gln Val Glu Gln Asp Ala Gln Ala Leu Phe
 
185 190 195
Gln Ala Gly Glu Leu Lys Trp Gly Thr Asp Glu Glu Lys Phe Ile

200 205 210
Thr Ile Phe Gly Thr Arg Ser Val Ser His Leu Arg Lys Val Phe

215 220 225
Asp Lys Tyr Met Thr Ile Ser Gly Phe Gln Ile Glu Glu Thr Ile

230 235 240
Asp Arg Glu Thr Ser Gly Asn Leu Glu Gln Leu Leu Leu Ala Val

245 250 255
Val Lys Ser Ile Arg Ser Ile Pro Ala Tyr Leu Ala Glu Thr Leu

260 265 270
Tyr Tyr Ala Met Lys Gly Ala Gly Thr Asp Asp His Thr Leu Ile

275 280 285
Arg Val Met Val Ser Arg Ser Glu Ile Asp Leu Phe Asn Ile Arg

290 295 300
Lys Glu Phe Arg Lys Asn Phe Ala Thr Ser Leu Tyr Ser Met Ile

305 310 315
Lys Gly Asp Thr Ser Gly Asp Tyr Lys Lys Ala Leu Leu Leu Leu

320
Cys Gly Glu Asp Asp *
 entspricht, wobei XX für Glu oder Asp steht und gegebenenfalls an Position 1 das Methionin abgespalten ist und das Alanin an Position 2 gegebenenfalls blockiert ist und/oder wobei gegebenenfalls Aggregationen durch beispielsweise intermolekulare Disulfidbrücken zwischen den Cysteinen an Position 316 vorliegen oder seinen biologisch aktiven Varianten oder Derivaten entspricht.

## Patentansprüche (Patentansprüche für folgende(n) Vertragsstaat(en): ES, GR)

1. Verfahren zur Herstellung eines Arzneimittels zur Vermeidung der Metastasierung von Tumoren, dadurch gekennzeichnet, daß ein Antikoagulanz aus der Gruppe der Annexine und seiner natürlich vorkommenden oder synthetisch oder gentechnisch hergestellten Derivate oder Analoga oder ein Salz eines solchen Antikoagulanz in einen pharmakologisch inerten Trägerstoff eingebracht wird.

2. Verfahren gemäß Anspruch 1, dadurch gekennzeichnet, daß das Antikoagulanz der Formel 
1                   5                   10                  15
Met Ala Gln Val Leu Arg Gly Thr Val Thr Asp Phe Pro Gly Phe

                    20                  25                  30
Asp Glu Arg Ala Asp Ala XX  Thr Leu Arg Lys Ala Met Lys Gly

                    35                  40                  45
Leu Gly Thr Asp Glu Glu Ser Ile Leu Thr Leu Leu Thr Ser Arg

                    50                  55                  60
Ser Asn Ala Gln Arg Gln Glu Ile Ser Ala Ala Phe Lys Thr Leu

                    65                  70                  75
Phe Gly Arg Asp Leu Leu Asp Asp Leu Lys Ser Glu Leu Thr Gly

                    80                  85                  90
Lys Phe Glu Lys Leu Ile Val Ala Leu Met Lys Pro Ser Arg Leu

                    95                  100                 105
Tyr Asp Ala Tyr Glu Leu Lys His Ala Leu Lys Gly Ala Gly Thr
 
110 115 120
Asn Glu Lys Val Leu Thr Glu Ile Ile Ala Ser Arg Thr Pro Glu

125 130 135
Glu Leu Arg Ala Ile Lys Gln Val Tyr Glu Glu Glu Tyr Gly Ser

140 145 150
Ser Leu Glu Asp Asp Val Val Gly Asp Thr Ser Gly Tyr Tyr Gln

155 160 165
Arg Met Leu Val Val Leu Leu Gln Ala Asn Arg Asp Pro Asp Ala

170 175 180
Gly Ile Asp Glu Ala Gln Val Glu Gln Asp Ala Gln Ala Leu Phe

185 190 195
Gln Ala Gly Glu Leu Lys Trp Gly Thr Asp Glu Glu Lys Phe Ile

200 205 210
Thr Ile Phe Gly Thr Arg Ser Val Ser His Leu Arg Lys Val Phe

215 220 225
Asp Lys Tyr Met Thr Ile Ser Gly Phe Gln Ile Glu Glu Thr Ile

230 235 240
Asp Arg Glu Thr Ser Gly Asn Leu Glu Gln Leu Leu Leu Ala Val
 
245 250 255
Val Lys Ser Ile Arg Ser Ile Pro Ala Tyr Leu Ala Glu Thr Leu

260 265 270
Tyr Tyr Ala Met Lys Gly Ala Gly Thr Asp Asp His Thr Leu Ile

275 280 285
Arg Val Met Val Ser Arg Ser Glu Ile Asp Leu Phe Asn Ile Arg

290 295 300
Lys Glu Phe Arg Lys Asn Phe Ala Thr Ser Leu Tyr Ser Met Ile

305 310 315
Lys Gly Asp Thr Ser Gly Asp Tyr Lys Lys Ala Leu Leu Leu Leu

320
Cys Gly Glu Asp Asp *
 entspricht, wobei XX für Glu oder Asp steht und gegebenenfalls an Position 1 das Methionin abgespalten ist und das Alanin an Position 2 gegebenenfalls blockiert ist und/oder wobei gegebenenfalls Aggregationen durch beispielsweise intermolekulare Disulfidbrücken zwischen den Cysteinen an Position 316 vorliegen oder seinen biologisch aktiven Varianten oder Derivaten entspricht.

3. Verfahren gemäß einem der Ansprüche 1 und 2, dadurch gekennzeichnet, daß der pharmakologisch inerte Träger einer wässrigen Lösung oder einer Lotion oder einem Gelee oder einer öligen Lösung oder einer Suspension oder einer fetthaltigen Salbe oder einer Emulsions-Salbe entspricht.

## Claims (Claims for the following Contracting State(s): AT, BE, CH, DE, DK, FR, GB, IT, LI, LU, NL, SE)

1. Use of an anticoagulant selected from the annexines and the naturally occurring derivatives or analogs thereof or those produced synthetically or by genetic engineering, for preparing a pharmaceutical composition for preventing tumour metastasis.

2. Use of an anticoagulant according to claim 1, wherein the anticoagulant corresponds to the formula 
1                   5                   10                  15
Met Ala Gln Val Leu Arg Gly Thr Val Thr Asp Phe Pro Gly Phe

                    20                  25                  30
Asp Glu Arg Ala Asp Ala XX  Thr Leu Arg Lys Ala Met Lys Gly

                    35                  40                  45
Leu Gly Thr Asp Glu Glu Ser Ile Leu Thr Leu Leu Thr Ser Arg

                    50                  55                  60
Ser Asn Ala Gln Arg Gln Glu Ile Ser Ala Ala Phe Lys Thr Leu

                    65                  70                  75
Phe Gly Arg Asp Leu Leu Asp Asp Leu Lys Ser Glu Leu Thr Gly

                    80                  85                  90
Lys Phe Glu Lys Leu Ile Val Ala Leu Met Lys Pro Ser Arg Leu

                    95                  100                 105
Tyr Asp Ala Tyr Glu Leu Lys His Ala Leu Lys Gly Ala Gly Thr

                    110                 115                 120
Asn Glu Lys Val Leu Thr Glu Ile Ile Ala Ser Arg Thr Pro Glu

                    125                 130                 135
Glu Leu Arg Ala Ile Lys Gln Val Tyr Glu Glu Glu Tyr Gly Ser
 
140 145 150
Ser Leu Glu Asp Asp Val Val Gly Asp Thr Ser Gly Tyr Tyr Gln

155 160 165
Arg Met Leu Val Val Leu Leu Gln Ala Asn Arg Asp Pro Asp Ala

170 175 180
Gly Ile Asp Glu Ala Gln Val Glu Gln Asp Ala Gln Ala Leu Phe

185 190 195
Gln Ala Gly Glu Leu Lys Trp Gly Thr Asp Glu Glu Lys Phe Ile

200 205 210
Thr Ile Phe Gly Thr Arg Ser Val Ser His Leu Arg Lys Val Phe

215 220 225
Asp Lys Tyr Met Thr Ile Ser Gly Phe Gln Ile Glu Glu Thr Ile

230 235 240
Asp Arg Glu Thr Ser Gly Asn Leu Glu Gln Leu Leu Leu Ala Val

245 250 255
Val Lys Ser Ile Arg Ser Ile Pro Ala Tyr Leu Ala Glu Thr Leu

260 265 270
Tyr Tyr Ala Met Lys Gly Ala Gly Thr Asp Asp His Thr Leu Ile

275 280 285
Arg Val Met Val Ser Arg Ser Glu Ile Asp Leu Phe Asn Ile Arg

290 295 300
Lys Glu Phe Arg Lys Asn Phe Ala Thr Ser Leu Tyr Ser Met Ile

305 310 315
Lys Gly Asp Thr Ser Gly Asp Tyr Lys Lys Ala Leu Leu Leu Leu

320
Cys Gly Glu Asp Asp *
 wherein XX denotes Glu or Asp and optionally at position 1 the methionine is cleaved and the alanine at position 2 is optionally blocked and/or there may optionally be aggregations caused, for example, by intermolecular disulphide bridges between the cysteines at position 316, or the biologically active variants or derivatives thereof.

## Claims (Claims for the following Contracting State(s): ES, GR)

1. Process for preparing a pharmaceutical composition for preventing tumour metastasis, characterised in that an anticoagulant selected from the annexines and the naturally occurring derivatives or analogs thereof or those produced synthetically or by genetic engineering, or a salt of an anticoagulant of this kind is incorporated in a pharmacologically inert carrier.

2. Process according to claim 1, characterised in that the anticoagulant corresponds to the formula 
1                   5                   10                  15
Met Ala Gln Val Leu Arg Gly Thr Val Thr Asp Phe Pro Gly Phe

                    20                  25                  30
Asp Glu Arg Ala Asp Ala XX  Thr Leu Arg Lys Ala Met Lys Gly

                    35                  40                  45
Leu Gly Thr Asp Glu Glu Ser Ile Leu Thr Leu Leu Thr Ser Arg

                    50                  55                  60
Ser Asn Ala Gln Arg Gln Glu Ile Ser Ala Ala Phe Lys Thr Leu

                    65                  70                  75
Phe Gly Arg Asp Leu Leu Asp Asp Leu Lys Ser Glu Leu Thr Gly

                    80                  85                  90
Lys Phe Glu Lys Leu Ile Val Ala Leu Met Lys Pro Ser Arg Leu

                    95                  100                 105
Tyr Asp Ala Tyr Glu Leu Lys His Ala Leu Lys Gly Ala Gly Thr
 
110 115 120
Asn Glu Lys Val Leu Thr Glu Ile Ile Ala Ser Arg Thr Pro Glu

125 130 135
Glu Leu Arg Ala Ile Lys Gln Val Tyr Glu Glu Glu Tyr Gly Ser

140 145 150
Ser Leu Glu Asp Asp Val Val Gly Asp Thr Ser Gly Tyr Tyr Gln

155 160 165
Arg Met Leu Val Val Leu Leu Gln Ala Asn Arg Asp Pro Asp Ala

170 175 180
Gly Ile Asp Glu Ala Gln Val Glu Gln Asp Ala Gln Ala Leu Phe

185 190 195
Gln Ala Gly Glu Leu Lys Trp Gly Thr Asp Glu Glu Lys Phe Ile

200 205 210
Thr Ile Phe Gly Thr Arg Ser Val Ser His Leu Arg Lys Val Phe

215 220 225
Asp Lys Tyr Met Thr Ile Ser Gly Phe Gln Ile Glu Glu Thr Ile

230 235 240
Asp Arg Glu Thr Ser Gly Asn Leu Glu Gln Leu Leu Leu Ala Val

245 250 255
Val Lys Ser Ile Arg Ser Ile Pro Ala Tyr Leu Ala Glu Thr Leu

260 265 270
Tyr Tyr Ala Met Lys Gly Ala Gly Thr Asp Asp His Thr Leu Ile

275 280 285
Arg Val Met Val Ser Arg Ser Glu Ile Asp Leu Phe Asn Ile Arg

290 295 300
Lys Glu Phe Arg Lys Asn Phe Ala Thr Ser Leu Tyr Ser Met Ile
 
305 310 315
Lys Gly Asp Thr Ser Gly Asp Tyr Lys Lys Ala Leu Leu Leu Leu

320
Cys Gly Glu Asp Asp *
 wherein XX denotes Glu or Asp and optionally at position 1 the methionine is cleaved and the alanine at position 2 is optionally blocked and/or there may optionally be aggregations caused, for example, by intermolecular disulphide bridges between the cysteines at position 316, or the biologically active variants or derivatives thereof.

3. Process according to one of claims 1 and 2, characterised in that the pharmacologically inert carrier corresponds to an aqueous solution or a lotion or a gel or an oily solution or a suspension or a greasy ointment or an emulsified ointment.

## Revendications (Revendications pour l'(les) Etat(s) contractant(s) suivant(s): AT, BE, CH, DE, DK, FR, GB, IT, LI, LU, NL, SE)

1. Utilisation d'un anticoagulant du groupe des annexines et de ses dérivés ou analogues naturels ou préparés par synthèse ou par génie génétique pour la préparation d'un médicament pour éviter la formation de métastases de tumeurs.

2. Utilisation d'un anticoagulant selon la revendication 1, dans laquelle l'anticoagulant correspond à la formule 
1                   5                   10                  15
Met Ala Gln Val Leu Arg Gly Thr Val Thr Asp Phe Pro Gly Phe

                    20                  25                  30
Asp Glu Arg Ala Asp Ala XX  Thr Leu Arg Lys Ala Met Lys Gly

                    35                  40                  45
Leu Gly Thr Asp Glu Glu Ser Ile Leu Thr Leu Leu Thr Ser Arg

                    50                  55                  60
Ser Asn Ala Gln Arg Gln Glu Ile Ser Ala Ala Phe Lys Thr Leu

                    65                  70                  75
Phe Gly Arg Asp Leu Leu Asp Asp Leu Lys Ser Glu Leu Thr Gly

                    80                  85                  90
Lys Phe Glu Lys Leu Ile Val Ala Leu Met Lys Pro Ser Arg Leu

                    95                  100                 105
Tyr Asp Ala Tyr Glu Leu Lys His Ala Leu Lys Gly Ala Gly Thr

                    110                 115                 120
Asn Glu Lys Val Leu Thr Glu Ile Ile Ala Ser Arg Thr Pro Glu
 
125                 130                 135
Glu Leu Arg Ala Ile Lys Gln Val Tyr Glu Glu Glu Tyr Gly Ser

140                 145                 150
Ser Leu Glu Asp Asp Val Val Gly Asp Thr Ser Gly Tyr Tyr Gln

155                 160                 165
Arg Met Leu Val Val Leu Leu Gln Ala Asn Arg Asp Pro Asp Ala

170                 175                 180
Gly Ile Asp Glu Ala Gln Val Glu Gln Asp Ala Gln Ala Leu Phe

185                 190                 195
Gln Ala Gly Glu Leu Lys Trp Gly Thr Asp Glu Glu Lys Phe Ile

200                 205                 210
Thr Ile Phe Gly Thr Arg Ser Val Ser His Leu Arg Lys Val Phe

215                 220                 225
Asp Lys Tyr Met Thr Ile Ser Gly Phe Gln Ile Glu Glu Thr Ile

230                 235                 240
Asp Arg Glu Thr Ser Gly Asn Leu Glu Gln Leu Leu Leu Ala Val

245                 250                 255
Val Lys Ser Ile Arg Ser Ile Pro Ala Tyr Leu Ala Glu Thr Leu

260                 265                 270
Tyr Tyr Ala Met Lys Gly Ala Gly Thr Asp Asp His Thr Leu Ile

275                 280                 285
Arg Val Met Val Ser Arg Ser Glu Ile Asp Leu Phe Asn Ile Arg
 
290 295 300
Lys Glu Phe Arg Lys Asn Phe Ala Thr Ser Leu Tyr Ser Met Ile

305 310 315
Lys Gly Asp Thr Ser Gly Asp Tyr Lys Lys Ala Leu Leu Leu Leu

320
Cys Gly Glu Asp Asp *
 dans laquelle XX représente Glu ou Asp et la méthionine en position 1 est éventuellement clivée et l'alanine en position 2 est éventuellement bloquée et/ou dans laquelle il existe éventuellement des agrégations par exemple par des ponts disulfure intermoléculaires entre les cystéines en position 316 ou correspond à ses variantes ou dérivés biologiquement actifs.

## Revendications (Revendications pour l'(les) Etat(s) contractant(s) suivant(s): ES, GR)

1. Procédé de préparation d'un médicament pour éviter la formation de métastases de tumeurs, caractérisé en ce qu'un anticoagulant du groupe des annexines et de ses dérivés ou analogues naturels ou préparés par synthèse ou par génie génétique ou un sel d'un tel anticoagulant est incorporé dans un véhicule inerte du point de vue pharmacologique.

2. Procédé selon la revendication 1, caractérisé en ce que l'anticoagulant correspond à la formule 
1                   5                   10                  15
Met Ala Gln Val Leu Arg Gly Thr Val Thr Asp Phe Pro Gly Phe

                    20                  25                  30
Asp Glu Arg Ala Asp Ala XX  Thr Leu Arg Lys Ala Met Lys Gly

                    35                  40                  45
Leu Gly Thr Asp Glu Glu Ser Ile Leu Thr Leu Leu Thr Ser Arg

                    50                  55                  60
Ser Asn Ala Gln Arg Gln Glu Ile Ser Ala Ala Phe Lys Thr Leu

                    65                  70                  75
Phe Gly Arg Asp Leu Leu Asp Asp Leu Lys Ser Glu Leu Thr Gly

                    80                  85                  90
Lys Phe Glu Lys Leu Ile Val Ala Leu Met Lys Pro Ser Arg Leu

                    95                  100                 105
Tyr Asp Ala Tyr Glu Leu Lys His Ala Leu Lys Gly Ala Gly Thr
 
110 115 120
Asn Glu Lys Val Leu Thr Glu Ile Ile Ala Ser Arg Thr Pro Glu
125 130 135
Glu Leu Arg Ala Ile Lys Gln Val Tyr Glu Glu Glu Tyr Gly Ser
140 145 150
Ser Leu Glu Asp Asp Val Val Gly Asp Thr Ser Gly Tyr Tyr Gln
155 160 165
Arg Met Leu Val Val Leu Leu Gln Ala Asn Arg Asp Pro Asp Ala
170 175 180
Gly Ile Asp Glu Ala Gln Val Glu Gln Asp Ala Gln Ala Leu Phe
185 190 195
Gln Ala Gly Glu Leu Lys Trp Gly Thr Asp Glu Glu Lys Phe Ile
200 205 210
Thr Ile Phe Gly Thr Arg Ser Val Ser His Leu Arg Lys Val Phe
215 220 225
Asp Lys Tyr Met Thr Ile Ser Gly Phe Gln Ile Glu Glu Thr Ile
230 235 240
Asp Arg Glu Thr Ser Gly Asn Leu Glu Gln Leu Leu Leu Ala Val
245 250 255
Val Lys Ser Ile Arg Ser Ile Pro Ala Tyr Leu Ala Glu Thr Leu
260 265 270
Tyr Tyr Ala Met Lys Gly Ala Gly Thr Asp Asp His Thr Leu Ile
275 280 285
Arg Val Met Val Ser Arg Ser Glu Ile Asp Leu Phe Asn Ile Arg
 
290 295 300
Lys Glu Phe Arg Lys Asn Phe Ala Thr Ser Leu Tyr Ser Met Ile

305 310 315
Lys Gly Asp Thr Ser Gly Asp Tyr Lys Lys Ala Leu Leu Leu Leu

320
Cys Gly Glu Asp Asp *
 dans laquelle XX représente Glu ou Asp et la méthionine en position 1 est éventuellement clivée et l'alanine en position 2 est éventuellement bloquée et/ou dans laquelle il existe éventuellement des agrégations par exemple par des ponts disulfure intermoléculaires entre les cystéines en position 316 ou correspond à ses variantes ou dérivés biologiquement actifs.

3. Procédé selon l'une des revendications 1 et 2, caractérisé en ce que le véhicule inerte du point de vue pharmacologique correspond à une solution aqueuse ou à une lotion ou à une gelée ou à une solution huileuse ou à une suspension ou à un onguent contenant des corps gras ou à un onguent en émulsion.
